# EUROPEAN PATENT SPECIFICATION

(11) **EP 2 097 521 B1**
(45) Date of publication and mention of the grant of the patent: **14.11.2012**
(21) Application number: 07834204.5
(22) Date of filing: 22.11.2007
(51) Int. Cl.: C12N 9/90

(54) **FOOD GRADE THERMOPHILIC ARABINOSE ISOMERASE EXPRESSED FROM GRAS, AND TAGATOSE MANUFACTURING METHOD BY USING IT**
VON GRAS EXPRIMIERTE THERMOPHILE ARABINOSE-ISOMERASE MIT LEBENSMITTEL-QUALITÄTSSTUFE SOWIE TAGATOSE-HERSTELLUNGSVERFAHREN UNTER VERWENDUNG DAVON
ARABINOSE ISOMÉRASE THERMOPHILE DE CLASSE ALIMENTAIRE EXPRIMÉE À PARTIR DE GRAS, ET PROCÉDÉ DESTINÉ À FABRIQUER DU TAGATOSE UTILISANT CELLE-CI

(30) Priority: 27.11.2006 KR 20060117792
(43) Date of publication of application: 09.09.2009
(73) Proprietor: CJ CheilJedang Corporation, Jung-gu Seoul 100-095 (KR)
(72) Inventor: KIM, Seong-bo, Seoul 120-113 (KR); LEE, Young-mi, Seoul 140-132 (KR); PARK, Seung-won, Gyeonggi-do 446-911 (KR); KIM, Jung-hoon, Seoul 152-763 (KR); SONG, Sang-hoon, Incheon City 403-102 (KR); LEE, Kang-pyo, Seoul 131-140 (KR)
(74) Representative: HOFFMANN EITLE
(86) International application number: PCT/KR2007/005899
(87) International publication number: WO 2008/066280

(56) References cited:
- WO-A1-02/052021
- KR-A- 20040 058 544
- US-A- 6 057 135
- WESTERS LIDIA ET AL: "Bacillus subtilis as cell factory for pharmaceutical proteins: a biotechnological approach to optimize the host organism" BIOCHIMICA ET BIOPHYSICA ACTA, vol. 1694, no. 1-3, 11 November 2004 (2004-11-11), pages 299-310, XP002562716 ISSN: 0006-3002
- SRIVASTAVA P ET AL: "Gene expression systems in corynebacteria" PROTEIN EXPRESSION AND PURIFICATION, ACADEMIC PRESS, SAN DIEGO, CA, vol. 40, no. 2, 1 April 2005 (2005-04-01), pages 221-229, XP004781372 ISSN: 1046-5928
- CHEON JINA ET AL: "Characterization of L-Arabinose Isomerase in Bacillus subtilis, a GRAS Host, for the Production of Edible Tagatose" FOOD BIOTECHNOLOGY (NEW YORK), vol. 23, no. 1, 2009, pages 8-16, XP009127914 ISSN: 0890-5436
- LEE S.J. ET AL. APPLIED AND ENVIRONMENTAL MICROBIOLOGY vol. 71, no. 12, 2005, pages 7888 - 7896, XP002499984

<110> CJ Corporation
<120> Food grade thermophilic arabinose isomerase expr essed from GRAS and tagatose manufacturing method by usi ng it
<130> PA06-0313
<160> 4
<170> Kopat ent I n 1.71
<210> 1
   <211> 38
   <212> DNA
   <213> Artificial Sequence
<220>
   <223> primer 1
<400> 1
   t ct agaat ga t gct gt cat t acgt cct t at gaat t t t g 38
<210> 2
   <211> 35
   <212> DNA
   <213> Artificial Sequence
<220>
   <223> primer 2
<400> 2
   t ct agat t ac cgcccccgcc aaaacact t c gt t cc 35
<210> 3
   <211> 31
   <212> DNA
   <213> Artificial Sequence
<220>
   <223> primer 3
<400> 3
   cccgat at ca t gct gt cat t acgt cct t at g 31
<210> 4
   <211> 31
   <212> DNA
   <213> Artificial Sequence
<220>
   <223> primer 4
<400> 4
   tgcactgcag ttaccgcccc cgccaaaaca c 31

## Description

### Technical Field

The present invention relates to a thermophilic arabinose isomerase and a method of manufacturing tagatose using the same, and more precisely, a gene encoding arabinose isomerase originating from the thermophile *Geobacillus stearothermophilus* DSM22, a recombinant expression vector containing the gene, a method of preparing a food grade thermophilic arabinose isomerase from the recombinant GRAS (Generally Recognized As Safe) strain transformed with the said expression vector, and a method of preparing tagatose from galactose by using the said enzyme.

### Background Art

With the increasing interest in well-being or a healthy life, tagatose has been proposed as an alternative to sugar as it has less side effects and sugar is one of the major factors causing various adult diseases. Tagatose is the isomer of galactose and is known to have fructose-like physiochemical properties. Tagatose is a natural low-calorie sugar, and has recently been approved by the FDA in the USA as GRAS (Generally Recognized As Safe), so it is now allowed to be added as a sweetener to foods, beverages, health foods, diet additives, etc.

GRAS indicates a substance that is generally recognized as safe, which is judged by specialized people having enough experience and skills through scientific procedure and examination under the indicated conditions and purpose of use. GRAS is a unique system used only in the USA to evaluate the safety of foods and food chemical substances (under certain conditions), but it is recognized world-wide.

Tagatose is produced by either isomerization, which is a chemical method using a catalyst to produce an isomer, of galactose or a biological method using isomerase to convert galactose enzymatically.

One of the biological methods well-known to those in the art is to convert aldose or aldose derivatives into ketose or ketose derivatives by using an enzyme. The isomerization of galactose into tagatose using arabinose isomerase is generally carried out thermodynamically at high temperature and exhibits a proportionally high conversion rate. Therefore, developing an enzyme that works stably at high temperature and a method of preparing tagatose using the same are key techniques for the industrial application thereof based on the biological conversion of tagatose using an isomerase. By screening thermophile derived arabinose isomerases, an industrially applicable thermophilic isomerase has been tried, and efforts have also been made by many research teams to establish an isomerization process using the same.

In Korea, an enzymatic isomerization method using arabinose isomerase has been developed by Tong Yang Confectionery Corp. According to the method, an *E. coli* derived arabinose isomerase gene was mass-expressed in *E. coli* by recombinant technology. This recombinant isomerase was reacted at 30°C for 24 hours to convert galactose into tagatose, and at this time the conversion rate was 25%, indicating that both thermostability and transformation yield were very low (Korean Patent Application No. 99-16118). Professor Oh and his colleagues (Sejong University) succeeded in the mass-expression of arabinose isomerase originating from *Geobacillus stearothermophilus* in *E. coli* by recombinant technology, and based on that they proposed an isomerization procedure at high temperature to convert galactose into tagatose. Tong Yang Confectionery Corp. team separated a thermophilic isomerase from a hot spring area by screening the thermophilic microorganism library and then expressed it as an active form in a recombinant *E. coli* host to use galactose for the high temperature isomerization process. Similarly, CheBiGen Inc. also produced thermophilic isonerase originating from *Geobacillus dinitrificans* DBG-A1 in *E. coli* and developed a technique to produce tagatose by immobilization using the same.

The expression level of the isomerase originating from the *Geobacillus* microorganism is too low to be applied in industry. The production of tagatose using a thermophile derived arabinose isomerase still depends on the method of using a recombinant enzyme mass-expressed in recombinant *E. coli* or the isomerization of galactose into tagatose using a host containing the recombinant enzyme. However, this biotechnological production of tagatose using recombinant *E. coli* is not appropriate for the production of tagatose as a food material. To produce tagatose as a food additive, arabinose isomerase expressed in a host which is a GRAS microorganism appropriate for the mass-production of the same is essential.

Industrially applicable GRAS microorganisms for the production of a recombinant enzyme can be selected from a group consisting of *Bacillus* sp., *Corynebacterium* sp., and *Lactobacillus* sp. It is easy to manipulate the genes of *Bacillus* sp. and *Corynebacterium* sp. strains for industrialization and mass-culture, and they are highly stable under various conditions. In fact, among GRAS microorganisms, mostly *Bacillus* sp. and *Corynebacterium* sp. strains have been used as a host for the production of a recombinant enzyme.

The present inventors succeeded in expressing thermophilic arabinose isomerase originating from *Geobacillus* as an active form in GRAS microorganisms such as *Bacillus* sp. and *Corynebacterium* sp. strains. The present inventors further established a method to induce isomerization of galactose into tagatose at high concentration by using the said expressed GRAS recombinant enzyme.

### Disclosure of Invention

### Technical Problem

It is an object of the present invention to express arabinose isomerase originating from a thermophile as an active form from GRAS microorganisms and to provide a method of preparing food grade tagatose by isomerization of galactose.

### Technical Solution

The above object and other objects of the present invention can be achieved by the following embodiments of the present invention.

To achieve the above objects, the present inventors produced a food grade recombinant enzyme by introducing a thermophilic arabinose isomerase gene derived from the thermophile *Geobacillus stearothermophilus* into a GRAS microorganism, and produced tagatose from galactose thereby.

Hereinafter, the present invention is described in detail.

GRAS (Generally Recognized As Safe) microorganism of the present invention preferably include *Bacillus* sp. and *Corynebacterium* sp. strains, and *Corynebacterium glutamicum* KCTC 13032 and *Bacillus subtilis* 168 are more preferred.

The arabinose isomerase gene of the present invention preferably originates from a thermophile, and more preferably from *Geobacillus stearothermophilus* DSM22.

The arabinose isomerase gene of the present invention can be modified by those in the art using any conventional mutagenesis method such as directed evolution and site-directed mutagenesis. Thus, any host cells that have a certain level of homology with a GRAS host, for example at least 70% but preferably at least 80% and more preferably at least 90% homology with a GRAS host, a recombinant enzyme that is expressed as an active form in the host and any host cells containing the enzyme are all included in the criteria of the present invention.

Embodiments of the present invention also provide a vector containing a gene encoding the arabinose isomerase of the present invention. The vector of the present invention is a typical vector for cloning or expression. The vector is not limited to a specific one and any vector known to those in the art is acceptable.

In the present invention, a promoter which is active in *Bacillus* and *Corynebacterium* was used as the vector to express a thermophilic isomerase as an active form.

The promoter sequence used for the gene expression in the *Corynebacterium* has not been identified unlike other promoters used in industrial microorganisms such as *E*. *coli* or *Bacillus subtilis.* Herein, a strong promoter that originates from *Corynebacterium,* a popular industrial microorganism, and is able to be expressed in *E. coli* has been developed. Tac promoter is known as being one of the strongest promoters. Tac promoter is prepared by fusion of a sequence of the -35 region of the tryptophane operon promoter of *E*. *coli* with a sequence of the -10 region of the lactose operon promoter of *E. coli.* The promoter composed of CJ-1 in the present invention was confirmed to be more effective in expressing a target gene in *Corynebacterium* sp. bacteria cells than tac promoter (Korean Patent Publication No. 10-2006-0068505).

The *Bacillus* promoter of the present invention exhibits promoter activity not only in *Bacillus* sp. microorganisms but also in *Lactobacillus* sp. microorganisms such as *Lactobacillus* and *Bifidobacterium. Corynebacterium* promoter also exhibits promoter activity in both *Corynebacterium* sp. microorganisms and *Escherichia* sp. bacteria, and *E. coli* cells. In particular, the promoter of the present invention showed promoter activity twice as strong in *Escherichia* sp. bacteria cells as tac promoter did.

According to the present invention, a recombinant strain was prepared by transforming *Bacillus* and *Corynebacterium* with the vector, which was then cultured to provide food grade arabinose isomerase. The culture medium and conditions depended on the kind of host.

According to the present invention, tagatose can be prepared from galactose by using the recombinant arabinose isomerase.

### Brief Description of the Drawings

The application of the preferred embodiments of the present invention is best understood with reference to the accompanying drawings, wherein:

Fig. 1 is a schematic diagram illustrating the construction of the recombinant expression vector pHT01 -GSA1 containing a gene encoding the thermophilic arabinose isomerase originating from the *Geobacillus stearothermophilus* DSM 22 strain,

Fig. 2 is a schematic diagram illustrating the construction of the recombinant expression vector pCJ-1-GSAI containing a gene encoding the thermophilic arabinose isomerase originating from the *Geobacillus stearothermophilus* DSM 22 strain,

Fig. 3 is a graph illustrating the enzyme activity of the thermophilic arabinose isomerase generated in the recombinant *Bacillus* host cells. A indicates the enzyme activity measured in a crude enzyme solution obtained from the culture of the host cells *(Bacillus subtilis* 168) only, and B indicates the enzyme activity measured in a crude enzyme solution obtained from the culture of the recombinant *Bacillus* host cells (GSAIB-1) containing the shuttle vector harboring the arabinose isomerase gene,

Fig. 4 is a graph illustrating the optimum conditions for the expression of the recombinant strain GSAIB-1 and the optimum conditions for the enzyme activity,

Fig. 5 is a graph illustrating the growth of the recombinant strain GSAIC-1 in the optimum medium.

### Best Mode for Carrying Out the Invention

Practical and presently preferred embodiments of the present invention are illustrated as shown in the following examples.

However, it will be appreciated that those skilled in the art, on consideration of this disclosure, may make modifications and improvements within the spirit and scope of the present invention.

[Example]

In the preferred embodiment of the present invention, a gene encoding thermophilic arabinose isomerase originating from the hyperthermophile *Geobacillus stearothermophilus* DSM 22 was inserted into pCJ-1 (*E. coli- Corynebacterium* shuttle vector, Korean Patent Publication No. 10-2006-0068505) and pHT10 *(E.coli-Bacillus* shuttle vector, Mo Bi Tech., Goettingen, Germany). *Corynebacterium glutamicum* KCTC 13032 and *Bacillus subtilis* 168 were transfected with the above vectors, in which the said protein was finally over-expressed. The recombinant strains *Corynebacterium glutamicum* GSAIC-1 and *Bacillus subtilis* GSAIB-1 were cultured and cell extracts were obtained from each stage of the cell culture. The production activity of tagatose was determined by measuring the amount of active recombinant protein stage by stage. The optimally expressed resultant culture was separated and purified by cell lysis, heat treatment. The production activity of tagatose was confirmed by the measurement of the protein activity.

Example 1 : Cloning of the arabinose isomerase

*Geobacillus stearothermophilus* DSM 22 was cultured under aerobic conditions. Centrifugation was performed at 8,000xg for 10 minutes to recover the cultured cells. Genomic DNA was extracted from the obtained cells by using a Cell culture DNA Midi Kit (Qiagen, U.S.A.). Polymerase Chain Reaction (PCR) was performed with the genomic DNA by using oligonucleotides 5'-TCTAGAATGATGCTGTCATTACGTCCTTATGAATTTTG-3' (SEQ. ID. NO: 1) and 5'-TCTAGATTACCGCCCCCGCCAAAACACTTCGTTCC-3' (SEQ. ID. NO: 2) with the insertion of Xbal and BamHi restriction enzyme site sequences as primers. PCR product 1 was obtained by amplifying the 1494 bp DNA containing the arabinose isomerase geneoriginating from *Geobacillus stearothermophilus.* PCR was performed again with the genomic DNA by using oligonucleotides 5'-CCCGAT ATCATGCTGT-CATTACGTCCTTATG-3' (SEQ. ID. NO: 3) and 5'-TGCACTGCAGTTACCGCCCCCG CCAAAACAC-3' (SEQ. D. NO: 4) with the insertion of EcoRV and PstI restriction enzyme site sequences as primers. PCR product 2 was obtained by amplifying 1512 bp DNA containing the arabinose isomerase geneoriginating from *Geobacillus stearothermophilus.* To over-express the arabinose isomerase encoded by the above two amplified genes, shuttle vector pHT01 originating from *Bacillus* sp. and shuttle vector pCJ-1 orignating from *Corynebacterium* sp. were used. The shuttle vector pCJ-1 was introduced into *E. coli* DH5alpha, which was deposited at the Korean Culture Center of Microorganisms (KCCM), an International Depositary Authority (IDA), on November 6, 2004 (Accession No: KCCM-10611). The shuttle vector pHT01 was obtained from Mo Bi Tech Co. (PBS001) (Table 1).

Table 1

**[Table 1]**

| Vector | Promoter | Vector | Accession/distribution No. | Derived protein |
|---|---|---|---|---|
| pCJ-1 | Pcj1 | pECCG117 | KCCM-10611 | Heat-shock protein hsp60 |
| pHT01 | Pgrac | - | PBS001 | - |

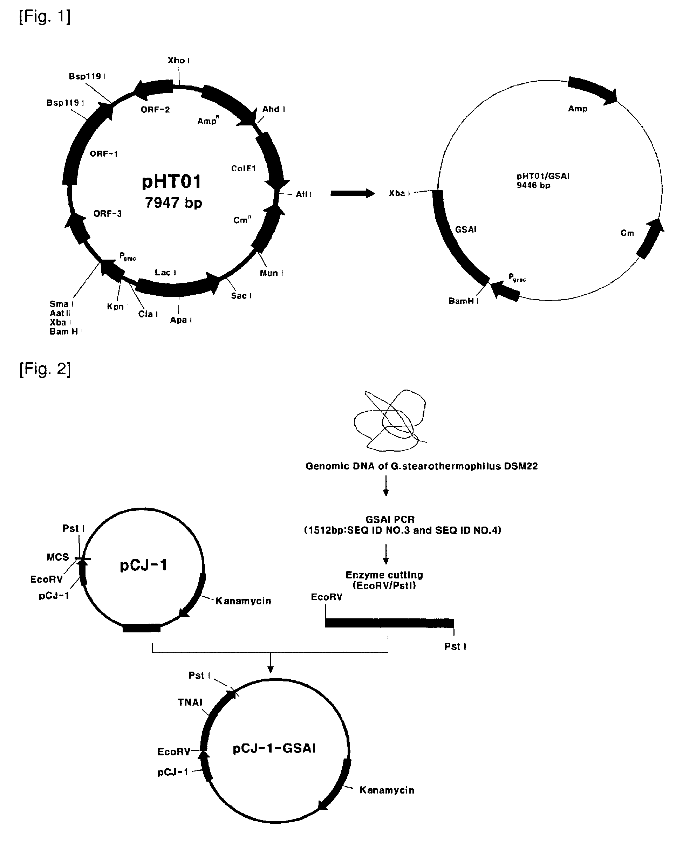

PCR product 1, digested by restriction enzymes XbaI and BamHI, was inserted into the shuttle vector pHT01, which was digested by the same enzymes, leading to the construction of the recombinant expression vector pHT01-GSAI (see Fig. 1). PCR product 2, digested with restriction enzymes EcoRV and PstI, was inserted into the shuttle vector pCJ-1, which was digested with the same restriction enzymes, leading to the construction of the recanbinant expression vector pCJ-1-GSA1 (see Fig. 2). *Corynebacterium glutamicum* KCTC 13032 and *Bacillus subtilis* 168 were transfected with the recombinant expression vectors pHT01-GSA1 and pCJ-1-GSA1 to prepare recombinant strains, which were named *Corynebacterium glutamicum* GSA1C-1 and *Bacillus subtilis* GSA1B-1'. The recombinant strains were deposited at the Korean Culture Center of Microorganisms (KCCM), an International Depositary Authority (IDA), addressed at #361-221, Hongje 1-Dong, Seodaemun-Gu, Seoul, Korea, on October 18, 2006 (Accession Nos: KCCM10789P and KCCM10788P).

Example 2: Expression of the recombinant arabinose isomerase in *Bacillus*

The recombinant strain *Bacillus subtilis* GSA1B-1 prepared in the above Example 1 (Accession No: KCCM10788P) was inoculated in LB medium (Bacto-trypton10 g/L, Bacto-yeast extract 5 g/L, NaCl 10 g/L) containing 20 µg/mℓ of chloramphenicol, followed by shaking-culture at 230 rpm and 37°C for 12 hours, resulting in the pre-culture solution. The pre-culture solution was inoculated in the main culture medium having the same composition at the concentration of 0.1%, followed by shaking-culture at 230 rpm and 37°C until OD₆₀₀reached 1 to induce the expression of the recombinant arabinose isomerase. To measure the enzyme activity of the expressed arabinose isomerase, the culture solution was centrifuged at 12,000xg for 10 minutes and cells were recovered. The cells were resuspended in 50 mM Tris-HCl (pH 8.2) buffer, followed by ultrasonification (170 Watt, cooling with ice at intervals of 1 second/2 minutes) to lyse the cells. Centrifugation was performed again at 12,000xg for 12 minutes to induce isomerization of galactose using the supernatant as a crude enzyme solution.

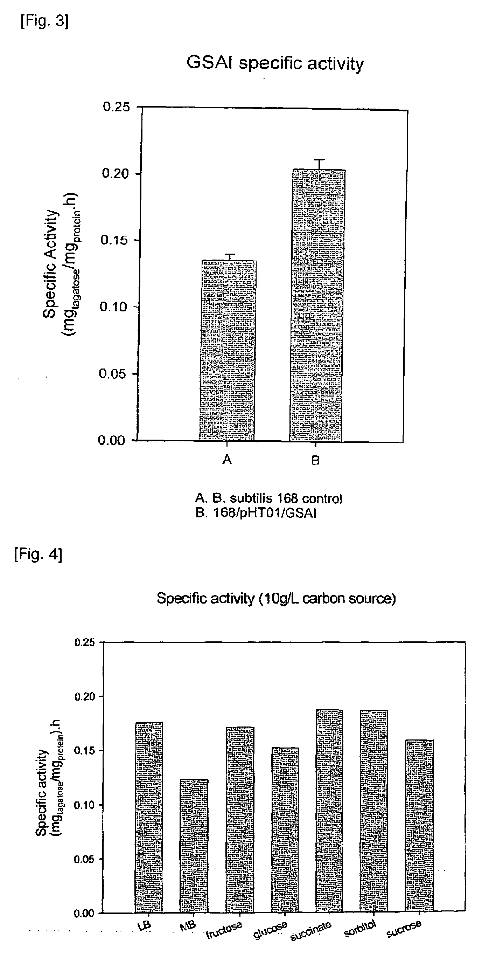

The isomerization of galactose was performed with a mixture of 25 µℓ of 100 mM galactose and 100µℓ of the crude enzyme solution as a substrate at 60°C for 1 hour. To measure the activity of galactose isomerization, 100 µℓ of the crude enzyme solution containing 40 mM of galactose as a substrate was mixed with 1 mℓ of reaction buffer (50 mM Tris-HCl, pH 7.0), followed by reaction at 65°C for 20 minutes. At that time, 5 mM of MgCl₂ and 1 mM of MnCl₂ were added to the reaction mixture. The activity of the isomerase was measured by the cystein-carbazol-sulfuric acid method (Dische, Z., and E. Borenfreund., A New Spectrophotometric Method for the Detection and Determination of Keto Sugars and Trioses, J. Biol. Chem., 192:583-587, 1951). The protein contained in the crude enzyme solution was quantified with a Bradford assay kit (Biorad, U.S.A.). As a result, the isomerase activity was 0.2045±0.0078 (mg-tagatose/mg-protein·h), indicating that the product of galactose isomerization, tagatose, was successfully generated (Fig. 3).

To provide optimum conditions for enzyme expression according to the composition of each culture solution, MB (Bacto-trypton 10 g/L, Bacto-yeast extract 5 g/L, NaCl 10 g/L, Soytone 10 g/L) and LB (Bacto-trypton 10g/L, Bacto-yeast extract 5 g/L, NaCl 10g/L) media, which have been generally used for the culture of *Bacillus,* were used as basic media. The recombinant strain GSAIB-1 was inoculated in LB medium (Bacto-trypton 10 g/L, Bacto-yeast extract 5g/L, NaCl 10g/L) containing chlorampenicol at the concentration of 20 µg/mℓ, followed by shaking-culture at 230 rpm and 37°C in a shaking incubator for 12 hours. The culture solution was used as a pre-culture solution. The pre-culture solution was inoculated in LB medium containing 10 g/L of carbon source (fructose, glucose, succinate, sorbitol or sucrose) and MB medium (Bacto-trypton 10 g/L, Bacto-yeast extract 5 g/L, NaCl 10 g/L, soytone 10 g/L) at the concentration of 0.1%, followed by shaking-culture at 230 rpm and 37°C in a shaking incubator until OD₆₀₀ reached 1, which suggests that the expression of the recombinant arabinose isomerase was induced. The expression level of the enzyme in MB medium was 30% lower than that in LB medium. Protein expression patterns according to the different carbon sources added were also investigated, for which industrially acceptable carbon sources such as glucose, fructose, succinate, sorbitol and sucrose were added to LB medium respectively by 10 g/L. The expression level of the enzyme was investigated. As a result, the enzyme expression was slightly increased with the addition of succinate or sorbitol (Fig. 4).

Example 3: Expression of the recombinant arabinose isomerase in *Corynebacterium*

The recombinant strain *Corynebacterium glutamicum* GSAIC-1 (Accession No: KCCM10789P) prepared in Example 1 was inoculated in MB medium (Bacto-trypton 10 g/L, Bacto-yeast extract 5 g/L, NaCl 10 g/L, Soytone 5 g/L) containing 10 µg/mℓ of kanamycin, followed by shaking-culture at 200 rpm and 30°C in a shaking incubator for 24 hours to prepare a pre-culture solution. The obtained pre-culture solution was inoculated in a main culture mediun at the concentration of 1%, followed by shaking-culture at 200 rpm and 30°C in a shaking incubator until OD₆₀₀ reached 0.1 to induce expression of the recombinant arabinose isomerase. To measure the enzyme activity of the expressed arabinose isomerase, the culture solution was centrifuged at 8000xg for 10 minutes and cells were recovered. The cells were resuspended in 50 mM Tris-HCl (pH 7.0) buffer solution, followed by ultrasonification to lyse the cells. Centrifugation was performed again at 8000xg for 12 minutes to induce isomerization of galactose using the supernatant as a crude enzyme solution. The protein included in the crude enzyme solution was quantified with a Bradford assay kit (Biorad, U.S.A.). As a result, the isomerase activity was 1.387 (mg-tagatose/mg-protein·h), indicating that the product of galactose isomerization, tagatose, was successfully generated.

To optimize the expression of arabinose isomerase in the recombinant strain *Corynebacterium* GSAIC-1, the recombinant strain was inoculated in MB medium (Bacto-trypton 10 g/L, Bacto-yeast extract 5 g/L, NaCl 10 g/L, Soytone 5 g/L) containing 10 µg/mℓ of kanamycin at the concentration of OD₆₀₀ = 0.6, resulting in the preparation of a pre-culture solution. The growth of the *Corynebacterium* strain in the two basic media for the culture, MB medium (Bacto-trypton 10 g/L, Bacto-yeast extract 5 g/L, NaCl 10 g/L, Soytone 5 g/L) and the modified medium (Bacto-peptone 10 g/L, Bacto-yeast extract 5 g/L, NaCl 2.5 g/L, Beef extract 5 g/L), was investigated. Temperature dependent (25°C, 30°C, 37°C), pH dependent, glucose (carbon source) and sucrose concentration dependent growths in the two media were compared. In addition, the growths under stationary and aerobic conditions were also compared. The growths under the various conditions and the expression levels of the enzyme thereby were measured every hour to judge the optimum expression conditions for mass-production of the recombinant arabinose isomerase (Tables 2 and 3).

Table 2

**[Table 2]**

| | Modified medium | MB medium | | | | |
|---|---|---|---|---|---|---|
| | Aerobic | Stationary | Aerobic | 25 °C | 30 °C | 37 °C |
| OD₆₀₀ | 5.66 | 9.0 | 9.34 | 7.44 | 9.34 | 4.64 |
| pH | 7.6 | 7.8 | 7.6 | 7.5 | 7.6 | 7.6 |
| Activity (mU/ml) | 21.859 | 29.008 | 31.639 | 30.826 | 31.639 | 25.833 |

Table 3

**[Table 3]**

| | 0% | Sucrose | | | | Glucose |
|---|---|---|---|---|---|---|
| | | 2.5% | 5% | 7.5% | 10% | 10% |
| OD₆₀₀ | 9.68 | 9.7 | 11.84 | 10.36 | 10.14 | 5.76 |
| pH | 7.6 | 4.7 | 4.4 | 4.4 | 4.6 | 4.4 |
| Activity (mU/ml) | 31.6 | 56.979 | 41.764 | 51.198 | 46.003 | 23.236 |
| Activity (mU/ml) | 31.6 | 56.979 | 41.764 | 51.198 | 46.003 | 23.236 |

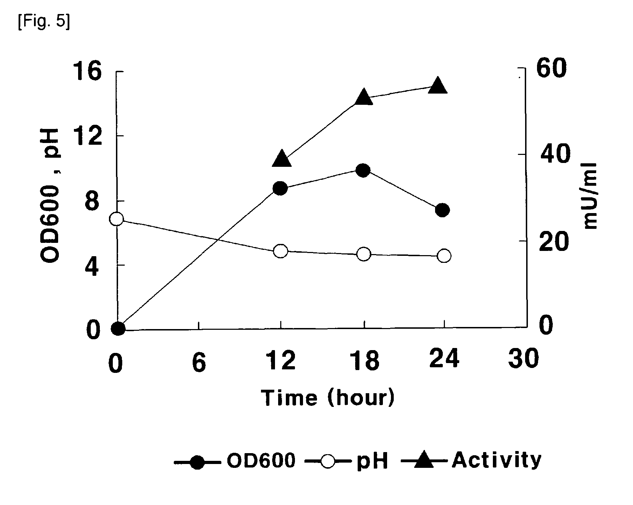

To measure the enzyme activity of the recombinant arabinose isomerase, the enzyme was treated and quantified in the same manner as described in Example 2. When cells were cultured at 30°C under aerobic conditions with the addition of 2.5% sucrose, tagatose showed approximately 57.0 mU/ml of enzyme activity which is 1.8 fold higher than that observed in the standard culture (31.6 mU/ml), suggesting that the enzyme activity increased with the increase in the growth of the cells. The cell growth results in the optimum medium are shown in Fig. 5.

Example 4: Separation and purification of the recombinant arabinose isomerase

2 L culture of the recombinant strain was performed under the optimum culture conditions determined in the above Example 3. The culture solution was centrifuged at 8000xg for 10 minutes and cells were recovered. The cells were resuspended in 50 mM Tris-HCl (pH 7.0) buffer, and used for the protein purification. The cell suspension progressed to cell lysis using a high pressure cell homogenizer T-series (4.0kW; Cbnstant systems, UK), followed by heat-treatment at 80°C for 20 minutes. Centrifugation was performed at 10,000xg for 10 minutes to separate the expressed recombinant thermophilic arabinose isomerase. The separated recombinant enzyme solution was filtered by ultrafiltration (MW: 10,000; Sartorius, U.S.A.). The filtrate was used for the subsequent experiments.

### Industrial Applicability

As explained hereinbefore, the present inventors confirmed that arabinose isomerase originating from the thermophilic microorganism *Geobacillus* in the present invention was successfully and stably expressed in GRAS microorganisms *Corynebacterium* sp. strain and *Bacillus* sp. strain. Accordingly, the present invention provides an active recombinant enzyme and a method of preparing tagatose which contains the step of an efficient immobilizing continuous reaction using the same. It is essential for a food additive to be safe, particularly in the production of biotechnological food using a microorganism enzyme. According to the present invention, arabinose isomerase originating from the *Geobacillus* sp. strain was confirmed to be of a safe food grade, so that it could be expressed and consecutively applied to industrialization.
## Claims

1. A method for preparing a food grade thermophilic arabinose isomerase comprising the steps of:
preparing a recombinant vector containing a gene encoding thermophilic arabinose isomerase originating from the *Geobacillus stearothermophilus* DSM 22 by inserting said gene into the shuttle vector pCJ-1 or pHT01, and
producing the thermophilic arabinose isomerase from *Corynebacterium* or *Bacillus* which is transfected with the recombinant vector.

2. The method for preparing a food grade thermophilic arabinose isomerase according to claim 1, wherein the *Bacillus* is *Bacillus subtilis* GSAIB-1 (Accession No. KCCM 10788P).

3. The method for preparing a food grade thermophilic arabinose isomerase according to claim 1, wherein the *Corynebacterium* is Corynebacterium glutamicum GSAIC-1 (Accession No. KCCM 10789P.

## Patentansprüche

1. Verfahren zur Herstellung einer thermophilen Arabinoseisomerase in Lebensmittelqualität, umfassend die Schritte:
Herstellen eines rekombinanten Vektors, welcher ein Gen enthält, welches für thermophile Arabinoseisomerase kodiert, welche vom Geobacillus stearothermophilus DSM 22 stammt, indem das Gen in den Shuttle-Vektor pCJ-1 oder pHT01 eingefügt wird und
Herstellen der thermophilen Arabinoseisomerase durch Corynebacterium oder Bacillus, transfiziert mit dem rekombinanten Vektor.

2. Verfahren zur Herstellung einer thermophilen Arabinoseisomerase in Lebensmittelqualität gemäß Anspruch 1, wobei das Bacillus Bacillus subtilis GSAIB-1 ist (Hinterlegungsnr. KCCM 10788P).

3. Verfahren zur Herstellung einer thermophilen Arabinoseisomerase in Lebensmittelqualität gemäß Anspruch 1, wobei das Corynebacterium Corynebacterium glutamicum GSAIC-1 ist (Hinterlegungsnr. KCCM 10789P)

## Revendications

1. Procédé de préparation d'une arabinose isomérase thermophile de classe alimentaire comprenant les étapes consistant :
à préparer un vecteur recombiné contenant un gène codant pour l'arabinose isomérase thermophile provenant de la Geobacillus stearothermophilus DSM 22 en insérant ledit gène dans le vecteur navette pCJ-1 ou pHT01, et
à produire l'arabinose isomérase thermophile à partir de la Bacillus ou de la Corynebacterium qui est transfectée avec le vecteur recombiné.

2. Procédé de préparation d'une arabinose isomérase thermophile de classe alimentaire selon la revendication 1, dans lequel la Bacillus est la Bacillus subtilis GSAIB-1 (n° d'entrée KCCM 10788P).

3. Procédé de préparation d'une arabinose isomérase thermophile de classe alimentaire selon la revendication 1, dans lequel la Corynebacterium est la Corynebacterium glutamicum GSAIC-1 (n° d'entrée KCCM 10789P).
